(19) Europäisches Patentamt
European Patent Office
Office européen des brevets

(11) EP 0 808 845 B1

(12) **FASCICULE DE BREVET EUROPEEN**

(45) Date de publication et mention de la délivrance du brevet:
**17.10.2001 Bulletin 2001/42**

(51) Int Cl.$^7$: **C07J 9/00**, A61K 31/575

(21) Numéro de dépôt: **97401111.6**

(22) Date de dépôt: **21.05.1997**

(54) **Nouveaux stéroides 1 ou 6-hydroxylés, leur procédé de préparation, leur application à titre de médicaments et les compositions pharmaceutiques les renfermant**

Neue 1- oder 6-hydroxylierte Steroide, ein Verfahren zu ihrer Herstellung, ihre Verwendung als Medikamenten und pharmazeutische Präparate davon

New 1- or 6-hydroxylated steroids, a process for their production, their use as medicaments and pharmaceutical compositions containing them

(84) Etats contractants désignés:
**AT BE CH DE DK ES FI FR GB GR IE IT LI LU NL PT SE**

(30) Priorité: **22.05.1996 FR 9606350**

(43) Date de publication de la demande:
**26.11.1997 Bulletin 1997/48**

(73) Titulaire: **Aventis Pharma S.A.**
**92160 Antony (FR)**

(72) Inventeurs:
- **Biton, Jacques**
  **60610 La Croix Saint Ouen (FR)**
- **Azerad, Robert**
  **91130 Ris Orangis (FR)**
- **Marchandeau, Jean-Pierre**
  **77410 Annet Sur Marne (FR)**
- **Lacroix, Isabelle**
  **94600 Choisy Le Roi (FR)**

(56) Documents cités:
**EP-A- 0 007 823** **US-A- 3 546 259**

- **CHEMICAL ABSTRACTS, vol. 080, no. 21, 27 mai 1974 Columbus, Ohio, US; abstract no. 116524, NIKOLAICHUK S P: "Biological activity of 6.alpha.-methoxyprogesterone" XP002019367 & FARMAKOL. TOKSIKOL., vol. 8, 1973, pages 88-90,**
- **CHEMICAL ABSTRACTS, vol. 082, no. 25, 23 juin 1975 Columbus, Ohio, US; abstract no. 165103, OKADA H ET AL: "Steroid specificity of the progestogen receptor of rabbit uterus. II" XP002019368 & NIPPON NAIBUMPI GAKKAI ZASSHI, vol. 50, no. 12, 1974, pages 1543-1547,**
- **SEELEY D H ET AL: "Molecular interactions of progesterone analogs with rabbit uterine cytoplasmic receptor" JOURNAL OF BIOLOGICAL CHEMISTRY, vol. 257, no. 22, 1982, MD US, pages 13359-13366, XP002019365**
- **MCGUIRE J L ET AL: "Interaction between steroids and a uterine progestogen specific binding macromolecule" BIOCHEMISTRY, vol. 13, no. 2, 1974, EASTON, PA US, pages 319-322, XP002019366**
- **F. KOBAYASHI ET AL: "Inhibitory and Facilitatory Effects of Steroids on the Release of Luteinising Hormone in the Rat" ENDOCRINOLOGIA JAPONICA, vol. 16, no. 5, octobre 1969, pages 493-499, XP000610857**
- **H. E. SMITH ET AL: "Binding of Steroids to Progesterone Receptor Proteins in Chick Oviduct and Human Uterus" JOURNAL OF BIOLOGICAL CHEMISTRY, vol. 249, no. 18, 25 septembre 1974, MD US, pages 5924-5932, XP000610861**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 808 845 B1

## Description

La présente invention a pour objet de nouveaux composés stéroïdes 1 ou 6-hydroxylés, leur procédé de préparation, leur application à titre de médicament et les compositions pharmaceutiques les renfermant.
L'invention a pour objet les composés de formule générale (I) :

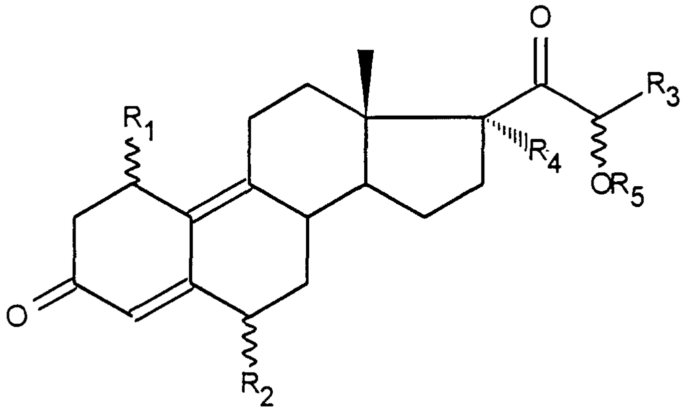

(I)

dans laquelle :

soit $R_1$ est un atome d'hydrogène et $R_2$ est un radical $OR_6$,
soit $R_1$ est un radical $OR_6$ et $R_2$ est un atome d'hydrogène, $R_6$ représentant un radical acyle renfermant de 1 à 12 atomes de carbone, éventuellement substitué par un ou plusieurs atomes d'halogène, ou un atome d'hydrogène,
$R_3$ est un radical alkyle renfermant de 1 à 12 atomes de carbone,
$R_4$ est un radical alkyle renfermant de 1 à 4 atomes de carbone,
et $R_5$, identique ou différent de $R_6$ possède les mêmes valeurs que $R_6$, les traits ondulés indiquant que $R_1$ ou $R_2$ sont en position α ou β et $OR_5$ est en position 21R ou 21S.

Par composé de formule générale (I), on désigne tous les isomères possibles pris individuellement ou en mélange.
Par radical alkyle renfermant de 1 à 12 atomes de carbone, on entend les radicaux suivants : méthyle, éthyle, propyle, isopropyle, butyle, isobutyle, tert-butyle, n-pentyle, n-hexyle, 2-méthylpentyle, 2,3-diméthylbutyle, n-heptyle, 2-méthylhexyle, 2,2-diméthylpentyle, 3,3-diméthylpentyle, 3-éthylpentyle, n-octyle, 2,2-diméthylhexyle, 3,3-diméthylhexyle, 3-méthyl-3-éthylpentyle, nonyle, 2,4-diméthylheptyle ou n-décyle.
Par radical alkyle renfermant de 1 à 4 atomes de carbone on entend les radicaux méthyle, éthyle, propyle, isopropyle, butyle, isobutyle ou tert-butyle.
Par radical acyle renfermant de 1 à 12 atomes de carbone on entend les radicaux suivants : formyle, acétyle, propionyle, butyryle, isobutyryle, valéryle, isovaléryle, succinyle, pivaloyle ou benzoyle.
Lorsqu'ils sont substitués par un atome d'halogène, il s'agit de préférence d'un atome de chlore ou d'iode ou de fluor. On peut citer les radicaux chloracétyle, dichloracétyle, trichloracétyle ou trifluoroacétyle.
Dans un premier groupe préféré, l'invention a pour objet les composés de formule (I) telle que définie précédemment dans laquelle $R_3$ et $R_4$ sont des radicaux méthyle.
Dans un deuxième groupe préféré l'invention a pour objet les composés de formule (I) telle que définie précédemment dans laquelle $R_1$ est un atome d'hydrogène et $R_2$ est un groupement hydroxyle.
Dans un troisième groupe préféré, l'invention a pour objet les composés de formule (I) telle que définie précédemment dans laquelle $R_1$ est un groupement hydroxyle et $R_2$ est un atome d'hydrogène.
Dans un quatrième groupe préféré, l'invention a pour objet les composés de formule (I) telle que définie précédemment dont les noms suivent :

- [17β-(S)]-1-hydroxy-17-(2-hydroxy-1-oxopropyl)-17-méthyl estra-4,9-dièn-3-one (produit A),
- [17β-(S)]-6-hydroxy-17-(2-hydroxy-1-oxopropyl)-17-méthyl estra-4,9-dièn-3-one (produit B),

ces composés pouvant être sous l'une quelconque de leur configuration stéréoisomère 1α-OH, 1β-OH, 6α-OH, 6β-OH, seuls ou en mélange.
L'invention a également pour objet un procédé de préparation des produits de formule (I).

Le procédé suivant l'invention, de préparation des composés de formule générale (I) est caractérisé en ce qu'on effectue une bioconversion du composé de formule générale (II) :

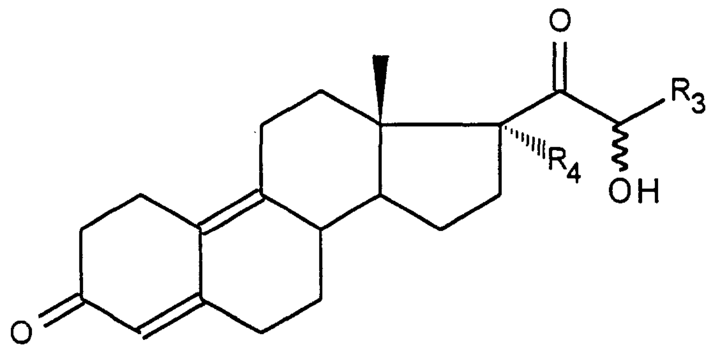

(II)

dans laquelle $R_3$, $R_4$ et les traits ondulés ont les mêmes définitions que celles données précédemment, avec une culture de champignons filamenteux, puis le cas échéant on procède à une séparation des produits 1-hydroxylés et 6-hydroxylés et/ou une estérification des groupements hydroxyles libres.

Parmi les champignons filamenteux connus de l'homme du métier, on choisit notamment les champignons suivants :

Pour la production du 1 OH et du 6 OH :

| | |
|---|---|
| Aspergillus terreus | MMP 2296 |
| Cuninghamella baineri | ATTC 9244 |
| Cuninghamella elegans | ATTC 26269 |
| Cuninghamella elegans | ATTC 36112 |
| Mortierella isabellina | NRRL 1757 |
| Mortierella isabellina | MMP 108 |
| Rhizopus arrhizus | ATCC 11145 |
| Thamnostylum piriforme | ATCC 8992 |
| Fusarium roseum | ATCC 14717 |
| Pour la production préférentielle du 1-OH : | |
| Cuninghamella baineri | ATTC 9244 |
| Mortierella isabellina | MMP 108 |
| Pour la production préférentielle du 6-OH : | |
| Mortierella isabellina | NRRL 1757 |
| Fusarium roseum | ATCC 14717 |

Origine des souches :

ATCC : American Type Culture Collection, Rockville, Md, USA.

MMP : Mycothèque du Muséum d'Histoire Naturelle, Paris,

NRRL : Northern Utilisation Research and Development Division, Peoria, Ill, USA.

La mise en oeuvre de l'hydroxylation s'effectue selon les méthodes qu'on applique couramment pour l'hydroxylation microbiologique des stéroides à l'aide de cultures de champignons (réf. : "Microbial conversions of steroids and alkaloids", H. Iizuka et A. Naito éditeurs, University of Tokyo Press, Springer Verlag, Berlin, 1981).

Ainsi, on détermine tout d'abord par voie analytique, en particulier par chromatographie en couche mince ou HPLC, dans des essais préalables généralement courants, les conditions de fermentation des plus favorables, comme par exemple le choix du milieu nutritif le plus favorable, du solvant de substrat approprié, de la concentration de substrat, des conditions techniques telles que température, aération, pH, et des périodes optimum pour la germination, l'addition de substrat et le contact du substrat avec le micro-organisme.

Il est apparu qu'il est avantageux de mettre en oeuvre des concentrations d'environ 40 à 2 000 mg de substrat par litre de milieu nutritif. On ajuste la valeur du pH de préférence à une valeur de l'ordre de 5 à 7. La température de culture est de l'ordre de 20 à 40°C, de préférence de 25 à 35°C. Pour l'aération, on amène environ 1 litre d'air par

minute par litre de bouillon de culture. La transformation du substrat est avantageusement suivie par analyse par chromatographie en couche mince d'échantillons extraits, ou par HPLC. En général après 24 à 144 heures il s'est formé des quantités suffisantes de stéroïde hydroxylé.

L'isolement, la séparation et la purification des produits du procédé s'effectuent d'une manière connue en soi. Par exemple, on peut extraire les produits du procédé avec un solvant organique tel que l'acétate d'éthyle, évaporer l'extrait, séparer et purifier les produits par chromatographie sur colonne.

L'invention a plus particulièrement pour objet un procédé tel que défini précédemment, dans lequel le champignon filamenteux est choisi parmi les souches suivantes :

| | |
|---|---|
| Cuninghamella baineri | ATTC 9244 |
| Mortierella isabellina | NRLL 1757 ou MMP 108 |
| Fusarium roseum | ATCC 14717. |

L'invention a plus particulièrement pour objet un procédé tel que défini précédemment, dans lequel le produit de formule (II) est la Trimégestone :

- [17β-(S)]-17-(2-hydroxy-1-oxopropyl)-17-méthyl-estra-4,9-dièn-3-one.

L'invention a tout particulièrement pour objet un procédé tel que défini précédemment, caractérisé en ce que le champignon filamenteux est le :

Cuninghamella baineri, le Mortierella isabellina ou le Fusarium roseum
et le produit de formule (II) est la Trimégestone, afin d'obtenir les dérivés 1-hydroxylé et 6-hydroxylé :

- [17β-(S)]-1-hydroxy-17-(2-hydroxy-1-oxopropyl)-17-méthyl estra-4,9-dièn-3-one,
- [17β-(S)]-6-hydroxy-17-(2-hydroxy-1-oxopropyl)-17-méthyl estra-4,9-dièn-3-one,

qui sont par la suite séparés.

L'invention a tout particulièrement pour objet un procédé tel que défini précédemment, caractérisé en ce que le champignon filamenteux est le Cuninghamella baineri ATCC 9244 et le produit de formule (II) est la Trimégestone, afin d'obtenir le dérivé 1-hydroxylé :

- [1α,17β-(S)]-1-hydroxy-17-(2-hydroxy-1-oxopropyl)-17-méthyl estra-4,9-dièn-3-one ou [1β,17β-(S)]-1-hydroxy-17-(2-hydroxy-1-oxopropyl)-17-méthyl estra-4,9-dièn-3-one, seuls ou en mélange.

L'estérification ultérieure éventuelle des groupes hydroxyles libres s'effectue suivant des procédés qu'on utilise couramment dans la chimie des stéroïdes pour l'estérification de groupes hydroxy secondaires et tertiaires. Comme procédé d'estérification approprié, on peut par exemple citer la réaction des stéroides avec des anhydrides ou des chlorures d'acide, en présence de catalyseurs basiques, tels que du bicarbonate de sodium ou de potassium, du carbonate de potassium, de l'hydroxyde de sodium ou de potassium, de la pyridine, de la lutidine, de la collidine ou de la 4-diméthylaminopyridine.

Les composés de formule générale (I) présentent d'intéressantes propriétés pharmacologiques, notamment une activité progestomimétique.

Ces composés ne présentent par ailleurs aucune affinité vis-à-vis des récepteurs estrogènes, androgènes, glucocorticoides et minéralocorticoides.

Les composés de formule générale (I) peuvent être utilisés à titre de médicaments dans le traitement des troubles gynécologiques dûs à une insuffisance lutéale :

- irrégularité menstruelle due à des troubles de l'ovulation,
- dysménorrhées,
- syndrome prémenstruel,
- mastodynie,
- hémorragies fonctionnelles et ménorragies des fibromes, troubles de la ménopause,
- stérilités,
- dystrophies ovariennes par mise au repos des ovaires, ou dans le traitement des tumeurs du sein et de l'utérus.

L'association des produits de formule générale (I) avec les estrogènes est nouvelle et constitue également

l'un des objets de l'invention ; elle trouve une application dans le traitement hormonal substitutif de la ménopause et en particulier dans la prévention ou le traitement de l'ostéoporose.

Parmi les estrogènes préférés on peut citer le 17β-estradiol et ses esters tels que l'estradiol valérate, cyprionate, décanoate et acétate, l'éthynyl estradiol, l'oestrone, l'estrogène "d'origine équine" tel que le Premarin® , ou une combinaison de ces composés.

L'association estrogène/produit de formule générale (I) trouve également son application à titre de contraceptif. L'estrogène sera alors de préférence l'éthynyl estradiol.

L'invention a donc pour objet les produits de formule générale (I) à titre de médicaments.

L'invention a également pour objet l'association estrogène/produits de formule générale (I) à titre de médicaments.

L'invention a plus particulièrement pour objet à titre de médicament, les produits A et B tels que décrits précédemment.

Les composés,

- [17β-(S)]-1-hydroxy-17- (2-hydroxy-1-oxopropyl)-17-méthyl estra-4,9-dièn-3-one,
- [17β-(S)]-6-hydroxy-17- (2-hydroxy-1-oxopropyl) -17-méthyl estra-4,9-dièn-3-one,

ces composés pouvant être sous l'une quelconque de leur configuration stéréoisomère 1α-OH, 1β-OH, 6α-OH, 6β-OH, seuls ou en mélange, peuvent donc être associés avec le 17β-estradiol et constituent ainsi un aspect particulier de l'invention.

L'invention a donc également plus particulièrement pour objet à titre de médicaments les associations telles que définies plus haut.

La posologie varie en fonction de l'affection à traiter et de la voie d'administration : elle peut varier par exemple de 1 mg à 1000 mg par jour chez l'adulte par voie orale selon l'indication.

L'invention s'étend aux compositions pharmaceutiques renfermant comme principe actif au moins un médicament tel que défini ci-dessus.

Les composés de formule (I) sont utilisés par voie digestive, parentérale ou locale, par exemple par voie percutanée. Ils peuvent être prescrits sous forme de comprimés simples ou dragéifiés, de gélules, de granulés, de suppositoires, d'ovules, de préparation injectables, de pommades, de crèmes, de gels, de microsphères, de nanosphères, d'implants, de patchs, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les composés de formule générale (II) utilisés comme produits de départ du procédé selon l'invention sont connus (brevet européen EP. 0007823).

Les souches utilisées lors du procédé selon l'invention sont connues.

Les exemples suivants illustrent l'invention sans toutefois la limiter.

**EXEMPLE 1 : Production/purification du 1α-OH-trimégestone**

On inocule avec une souche lyophilisée de Cuninghamella baineri (ATCC 9244) un milieu nutritif solide constitué, pour 1 litre d'eau distillée, de 20 g de glucose, 5 g de peptone pancréatique, 5 g d'extrait de levure, 5 g d'extrait de malt et 20 g de bactoagar. On incube le tout stérilement pendant 7 jours à 25°C, dans une étuve.

Au bout des 7 jours, les spores sont récoltées. Ces spores fraîches sont alors utilisées pour ensemencer 2 flacons d'Erlenmeyer de 2 litres qui contiennent chacun 800 ml d'une solution nutritive stérilisée pendant 30 minutes dans un autoclave.

Ce milieu nutritif contient : 10 g de corn steep, 2 g de $NaNO_3$, 0,5 g de $MgSO_4$, $7H_2O$, 0,02 g de $FeSO_4$, $7H_2O$, 0,5 g de KCl pour 900 ml d'eau distillée. On ajoute au moment de l'ensemencement une solution stérile de tampon Phosphate ($K_2HPO_4$, 2 g ; $KH_2PO_4$, 1 g dans 40 ml d'eau) et un complément nutritif sous la forme de 60 ml d'une solution aqueuse stérilisée contenant 30 g de glucose.

On laisse la culture se développer à 27°C, sous agitation à 200 tours/minute dans un secoueur rotatif. Après 65 h, on ajoute, dans chaque erlenmeyer, une solution de 400 mg de trimégestone dans 5 ml d'éthanol à 99 %.

Après transformation totale du substrat mis en oeuvre (72 heures de contact), le milieu d'incubation est filtré et ce filtrat est ensuite saturé par ajout d'un large excès de NaCl. La solution saturée en NaCl est alors extraite à trois reprises sous agitation par 250 ml d'acétate d'éthyle. Après séchage sur $MgSO_4$, on évapore l'extrait sous vide à une température de bain de 40°C et on obtient 965 mg d'une huile jaunâtre.

Cette huile est chromatographiée sur une colonne de gel de silice 60H (Merck, 70-230 Mesh) par un mélange

chlorure de méthylène/isopropanol (95:5). On obtient 5 fractions principales. La fraction de 209 mg contenant majoritairement du produit A est purifiée par chromatographie liquide haute pression semi préparative (250 x 21,2 mm), en phase inverse greffée C18, avec un mélange méthanol/eau (50/50).

Après évaporation sous vide du solvant à une température de bain de 40°C, on obtient 84 mg de [1α,17β-(S)]-1-hydroxy-17-(2-hydroxy-1-oxopropyl)-17-méthyl-estra-4,9-dièn-3-one (produit A), avec une pureté chromatographique supérieure à 96 %.
Analyses RMN $^1H$ et $^{13}C$ : voir tableaux 1 et 2.

**<u>EXEMPLE 2 :</u> Production/purification du 6β-OH-trimégestone**

On inocule avec une souche lyophilisée de Mortierella isabellina (NRRL 1757) un milieu nutritif solide constitué, pour 1 litre d'eau distillée, de 20 g de glucose, 5 g de peptone pancréatique, 5 g d'extrait de levure, 5 g d'extrait de malt et 20 g de bactoagar.

On incube le tout stérilement pendant 7 jours à 25°C, dans une étuve.

Au bout des 7 jours, les spores sont récoltées. Ces spores fraîches sont alors utilisées pour ensemencer 5 flacons d'Erlenmeyer de 240 ml qui contiennent chacun 100 ml d'une solution nutritive stérilisée pendant 30 minutes dans un autoclave.

Ce milieu nutritif contient : 10 g de corn steep, 2 g de $NaNO_3$, 0,5 g de $MgSO_4$, $7H_2O$, 0,02 g de $FeSO_4$, $7H_2O$, 0,5 g de KCl pour 900 ml d'eau distillée. On ajoute au moment de l'ensemencement une solution stérile de tampon Phosphate ($K_2HPO_4$, 2 g ; $KH_2PO_4$, 1 g dans 40 ml d'eau) et un complément nutritif sous la forme de 60 ml d'une solution aqueuse stérilisée contenant 30 g de glucose.

On laisse la culture se développer à 27°C, sous agitation dans un secoueur rotatif. Après 60-66 h, on ajoute, dans chaque erlenmeyer, une solution de 50 mg de trimégestone dans 1 ml d'éthanol à 99 %.

Après transformation totale du substrat mis en oeuvre (48 heures de contact), on filtre sur un filtre en tissu pour séparer le mycélium du bouillon de fermentation. Le mycélium est ensuite largement lavé à l'eau distillée. Le filtrat du lavage et le bouillon filtré de fermentation sont alors réunis. Puis on sature le mélange "filtrat de lavage/bouillon filtré" par ajout d'un large excès de NaCl.

La solution saturée en NaCl est alors extraite à trois reprises sous agitation par 250 ml d'acétate d'éthyle. Puis on évapore l'extrait sous vide à une température de bain de 40°C.

Pour nettoyer l'extrait de produit secondaires issus de la fermentation, on chromatographie sur une colonne de gel de silice 60H (Merck, 230-400 Mesh) à l'aide d'un mélange chlorure de méthylène/méthanol (95-5). Les fractions obtenues sont regroupées en une seule fraction, ayant un aspect huileux.

Finalement, on purifie par chromatographie liquide haute pression semi préparative (250 x 21,2 mm), en phase inverse greffée C18, avec un mélange méthanol/eau (60/40).

Après évaporation sous vide du solvant de la fraction intéressante, à une température de bain de 40°C, on obtient 21 mg de [6β,17β-(S)]-6-hydroxy-17-(2-hydroxy-1-oxopropyl) 17-méthyl-estra-4,9-dièn-3-one (produit B), avec une pureté chromatographique supérieure à 90 %.
Analyses RMN $^1H$ et $^{13}C$ : voir tableaux 1 et 2.

**<u>EXEMPLE 3</u> : Production/purification du 1α-OH-trimégestone et du 6β-OH-trimégestone**

On prépare cinq flacons d'Erlenmeyer de 500 ml qui contiennent chacun 90 ml d'une solution nutritive n° 1. Ce milieu nutritif contient : 10 g de corn steep liquor, 2 g de $NaNO_3$, 0,5 g de $MgSO_4$, $7H_2O$, 0,02 g de $FeSO_4$, $7H_2O$, 0,5 g de KCl et QSP 900 ml d'eau distillée. De même, on prépare un flacon Schott contenant une solution nutritive n° 2, comprenant : 10 g de $K_2HPO_4$, 5 g de $KH_2PO_4$ et QSP 200 ml d'eau déminéralisée. On prépare aussi un flacon Schott contenant une solution nutritive n° 3, comprenant 150 g de glucose et QSP 300 ml d'eau déminéralisée. Dans la pratique, dissoudre à chaud sous agitation forte dans 150 ml d'eau déminéralisée 150 g de glucose. Après dissolution complète ajuster le volume à 300 ml à l'éprouvette.

Les 5 erlenmeyers et les 2 flacons Schott sont stérilisés pendant 30 minutes à 121°C dans un autoclave.

Juste avant ensemencement des précultures, on ajoute stérilement, dans chaque Erlen contenant 90 ml de la solution nutritive n° 1, 4 ml de la solution N° 2 et 6 ml de la solution n° 3. Puis on inocule avec 1 ml d'inoculum congelé de la souche de Fusarium roseum (ATCC 14717).

Pendant 48 h, on agite les 5 erlenmeyers à 200 tours/ minute, à une température de 27°C dans un agitateur orbital, ayant une excentration de 2,5 cm.

Avec cette culture préalable, on inocule un fermenteur de 3 litres qui contient la solution nutritive A, à savoir : 30 g de corn steep liquor, 6 g de $NaNO_3$, 1,5 g de $MgSO_4$, $7H_2O$, 0,06 g de $FeSO_4$, $7H_2O$, 1,5 g de KCl et QSP 2650 ml d'eau déminéralisée. Le fermenteur est ensuite stérilisé à l'autoclave 45 minutes à 121°C.

On prépare ensuite une fiole d'introduction de 500 ml contenant 300 ml d'une solution B. Celle-ci est préparée

comme suit : dans un becher de 500 ml contenant un barreau magnétique, on place 150 ml d'eau distillée et on agite à chaud. Puis on introduit lentement 90 g de glucose. Après dissolution complète, on ajoute 6 g de $K_2HPO_4$ puis 3 g de $KH_2PO_4$. On ajuste le volume à 300 ml avec de l'eau distillée (à l'éprouvette). La fiole est ensuite stérilisée à l'autoclave 30 minutes à 121°C.

Avant l'ensemencement, on introduit stérilement les 300 ml de la solution B dans le fermenteur. Puis on inocule avec 50 ml de la préculture à l'aide d'une fiole d'inoculation. La fermentation est conduite à une température de 27°C, avec une pression de 0,3 bar, une agitation initiale de 200 rpm et une aération de 90 nl/h soit 0,50 vvm. La pression partielle en oxygène est régulée à 50 % et le pH régulé à 6,5 par de l'acide sulfurique 1M jusqu'à 28 h. Il évolue ensuite librement jusqu'à la fin de la bioconversion.

Au bout de 46 h, après la phase de croissance active, 6 g de trimégestone sont dissous dans 70 ml d'acétone puis ajoutés stérilement à la suspension cellulaire. La culture est ensuite poursuivie dans les mêmes conditions.

Après 96 h, la fermentation est arrêtée. On filtre alors sur un filtre en tissu pour séparer le mycélium du bouillon de fermentation. Le mycélium est ensuite largement lavé à l'eau distillée. Le filtrat du lavage et le bouillon filtré de fermentation sont alors réunis. La solution saturée en NaCl est alors extraite à trois reprises sous agitation par 500 ml d'acétate d'éthyle et une fois par 500 ml de chlorure de méthylène. Les fractions organiques sont regroupées et distillées à sec sous vide à une température de bain de 40°C.

On purifie l'extrait sec obtenu par chromatographie liquide haute pression préparative, en phase normale, sur silice sphérique 10 μ dans un mélange chlorure de méthylène/ méthanol (95-5).

On obtient plusieurs fractions, dont 2 contiennent le produit A et le produit B. Après évaporation sous vide du solvant, à une température de bain de 40°C, on obtient ainsi :

- le [1α,17β-(S)]-1-hydroxy-17-(2-hydroxy-1-oxopropyl)-17-méthyl-estra-4,9-dièn-3-one/17β-estradiol (produit A),
- et le [6β,17β-(S)]-6-hydroxy-17-(2-hydroxy-1-oxopropyl) 17-méthyl-estra-4,9-dièn-3-one/17β-estradiol (produit B) avec une pureté chromatographique supérieure à 90 %.

Analyses RMN $^1H$ et $^{13}C$ : voir tableaux 1 et 2.

Tableau 1 :

| RMN 1H des 2 métabolites de la trimégestone (250 MHz, $CDCl_3$ ; d en ppm, J en Hz). Ce tableau rassemble les résultats obtenus. La plupart des attributions décrites ont été effectuées après étude des corrélations 2D 1H-1H ou 1H-13C. | | | |
|---|---|---|---|
| | Trimégestone | 6β-OH Trimégestone Produit B | 1α-OH Trimégestone Produit A |
| 1α<br>et<br>1β | | 2,6 (m)<br>et<br>2,9 (m) | -<br>5,10<br>(br.s) |
| 2α<br>et<br>2β | | 2,5 (m) | 2,6 (m)<br>et<br>2,7 (m) |
| 4 | 5,67 (s) | 5,82 (s) | 5,76 (s) |
| 6α<br>et<br>6β | | 4,37 (dd)<br>J = 3,5 | 2,4 (m)<br>et<br>2,6 (m) |
| 7α<br>et<br>7β | | 1,5 (ddd)<br>et<br>2,1 (m) | 1,45 (m)<br>et<br>1,9 (m) |
| 8β | | 2,6 (m) | 2,3 (m) |
| 11α<br>et<br>11β | | 2,2 (dt)<br>et<br>2,8 (m) | 2,4 (m)<br>et<br>3,0 (dm) |
| 12α<br>et<br>12β | | 1,7 (m)<br>et<br>2,0 (m) | 1,7 (m)<br>et<br>2,05 (m) |

Tableau 1 : (suite)

| | Trimégestone | 6β-OH Trimégestone Produit B | 1α-OH Trimégestone Produit A |
|---|---|---|---|
| 14β | | 1,68 (m) | 1,80 (m) |
| 15α<br>et<br>15β | | 1,4 (m)<br>et<br>2,7 (m) | 1,4 (m)<br>et<br>2,7 (m) |
| 16a<br>et<br>16β | | 1,4 (m)<br>et<br>1,8 (m) | 1,4 (m)<br>et<br>1,8 (m) |
| 18 | 0,81 (s) | 0,84 (s) | 0,85 (s) |
| 19 | 1,16 (s) | 1,15 (s) | 1,16 (s) |
| 21 | 4,41 (q)<br>J = 6,4 | 4,40 (q)<br>J = 6,4 | 4,40 (q)<br>J = 6,4 |
| 22 | 1,31 (d)<br>J = 6,4 | 1,30 (d)<br>J = 6,4 | 1,31 (d)<br>J = 6,4 |

Tableau 2 :

| RMN 13C des 2 métabolites de la trimégestone (69,2 MHz, $CDCl_3$) | | | |
|---|---|---|---|
| Carbone n° | Trimégestone | 6β-OH Trimégestone Produit B | 1α-OH Trimégestone Produit A |
| 1 | 25,6 | 25,9 | 65,9 |
| 2 | 36,9 | 37,3 | 45,5 |
| 3 | 199,5 | 200,2 | 197,6 |
| 4 | 122,1 | 122,3 | 121,5 |
| 5 | 156,9 | 155,4 | 154,3 |
| 6 | 30,7 | 68,4 | 31,0 |
| 7 | 27,6 | 34,3 | 27,3 |
| 8 | 39,4 | 34,6 | 39,7 |
| 9 | 145,4 | 145,3 | 150,6 |
| 10 | 125,5 | 122,7 | 127,9 |
| 11 | 25,7 | 25,7 | 25,4 |
| 12 | 32,8 | 33,0 | 33,0 |
| 13 | 45,1 | 45,4 | 45,1 |
| 14 | 51,0 | 50,6 | 51,4 |
| 15 | 24,0 | 30,7 | 30,3 |
| 16 | 30,8 | 24,0 | 23,9 |
| 17 | 60,0 | 60,0 | 60,0 |
| 18 | 15,7 | 15,8 | 15,8 |
| 19 | 21,5 | 21,7 | 21,6 |
| 20 | 217,0 | 217,2 | 217,1 |
| 21 | 69,6 | 69,7 | 69,7 |
| 22 | 22,0 | 22,1 | 21,6 |

**Etude pharmacologique des produits de l'invention**

1 - Etude de l'activité des produits de l'invention sur les récepteurs hormonaux.

On utilise le récepteur humain recombinant (RGH, RPH, RAH, RMH, REH).

Récepteur progestérone humain (RPH) :

Le récepteur progestogène humain recombinant est obtenu par surexpression dans un système cellules d'insectes-Baculovirus, selon la méthodologie générale décrite par N.R. WEBB et al. (Journal of Methods in Cell and Molecular Biology, (1990) Vol 2 n° 4, 173-188) et dont l'application est décrite pour l'expression des récepteurs hormonaux humains, par exemple le récepteur glucocorticoide humain (G. SRINIVASAN et al. Molecular Endocrinology (1990) vol 4 n° 2 209-216).

On utilise le kit BaculoGold Transfection Kit (PharMingen, référence 21000K) pour insérer le fragment d'ADNc décrit par P. KASTNER et al. (The EMBO Journal (1990) vol 9 n° 5, 1603-1614), comprenant la région codante pour le récepteur progestogène humain et pour préparer le virus recombinant correspondant.

Le virus recombinant ainsi obtenu est utilisé pour exprimer le récepteur progestogène dans les cellules d'insectes SF9 (ATCC CRL1711), selon la méthodologie connue citée précédemment.

2 x $10^7$ à 2,5 x $10^7$ cellules SF9 sont cultivées dans un flacon "Falcon" de 175 $cm^2$ dans le milieu TNM-FH supplémenté avec 10 % de sérum de veau foetal (SVF) et avec 50 microgrammes/ml de gentamycine. Après infection puis incubation à 27°C pendant 40 à 42 heures, les cellules sont reprises dans 1 ml de tampon de lyse (1), lysées par 2 cycles de congélation-décongélation (-80°C/0°C) puis centrifugées à 4°C pendant 30 minutes à 20900 g. Le surnageant, contenant le récepteur progestogène humain recombinant est conservé dans l'azote liquide par quantité de 1 ml.

Le surnageant est dilué au moment de l'emploi avec du tampon Tris 10mM, saccharose 0,25 M, HCl pH 7,4 contenant 0,1 % de gélatine, puis incubé à 0°C pendant 24 heures avec une concentration constante (T) de 17α, 21-diméthyl 19-norpregna 4,9-dièn-3,20-dione tritié en présence de concentrations croissantes soit de progestérone froide (0-2500 x $10^{-9}$M), soit du produit froid à tester (1 à 25000 x $10^{-9}$M). La concentration de 17,21-diméthyl 19-nor-4,9-pregnane-3,20-dione tritié lié (B) est ensuite mesurée dans chaque incubat par la technique d'adsorption au charbon dextran.

Récepteur glucocorticoide humain (RGH) :

Un surnageant de cellules SF9 contenant le récepteur glucocorticoide humain recombinant est obtenu selon le procédé décrit ci-dessus pour le récepteur progestogène en utilisant le fragment d'ADNc décrit par S.M. HOLLENBERG et al. (Nature (1985) vol. 318 n° 19/26 635) comprenant la région codante pour le récepteur glucocorticoïde humain. Les cellules obtenues sont lysées dans le tampon de lyse (2).

Le surnageant est incubé à 0°C pendant 24 heures avec une concentration constante (T) de 11β,17β-dihydroxy-6,21-diméthyl pregna 1,4,6-trièn-20-yn-3-one tritié en présence de concentrations croissantes soit de dexaméthasone froide (0-1000 x $10^{-9}$M), soit du produit froid à tester (1 à 25000 x $10^{-9}$M). La concentration de 11β,17β-dihydroxy-6,21-diméthyl pregna 1,4,6-trièn-20-yn-3-one tritié lié (B) est ensuite mesurée dans chaque incubat par la technique d'adsorption au charbon dextran.

Récepteur oestrogène humain (REH) :

Un surnageant de cellules SF9 contenant le récepteur oestrogène humain recombinant est obtenu selon le procédé décrit ci-dessus pour le récepteur progestogène en utilisant le fragment d'ADNc décrit dans le vecteur d'expression HEGO par L. TORA et al. (The EMBO Journal (1989) vol. 8 n° 7 1981-1986), comprenant la région codante pour le récepteur oestrogène humain de "type sauvage" avec une glycine en position 400. Les cellules obtenues sont lysées dans le tampon de lyse (1).

Le surnageant est incubé à 0°C pendant 24 heures avec une concentration constante (T) d'oestradiol tritié en présence de concentrations croissantes soit d'oestradiol froid (0-1000 x $10^{-9}$M), soit du produit froid à tester (1 à 25000 x $10^{-9}$M). La concentration d'oestradiol tritié lié (B) est ensuite mesurée dans chaque incubat par la technique d'adsorption au charbon dextran.

Récepteur androgène humain (RAH) :

Un surnageant de cellules SF9 contenant le récepteur androgène humain recombinant est obtenu selon le procédé décrit ci-dessus pour le récepteur progestogène en utilisant le fragment d'ADNc comprenant la région codante

pour le récepteur androgène humain. Les cellules obtenues sont lysées dans le tampon de lyse (3).

Le surnageant est incubé à 0°C pendant un temps d'incubation de 24 heures avec une concentration constante (T) de testostérone tritiée en présence de concentrations croissantes, soit de testostérone froide (0 à 1000 x $10^{-9}$M), soit du produit à tester (1 à 25000 x $10^{-9}$M). La concentration de testostérone tritiée liée (B) est ensuite mesurée dans chaque incubat par la technique d'adsorption au charbon dextran.

<u>Récepteur minéralocorticoïde humain</u> (RMH) :

Un surnageant de cellules SF9 contenant le récepteur minéralocorticoide humain recombinant est obtenu selon le procédé mentionné ci-dessus pour le récepteur progestogène, en utilisant le fragment d'ADNc décrit par E. E. BAULIEU et al. (Proc. Natl. Acad. Sci. (1991) Vol. 88, 10681-10685) comprenant la région codante pour le récepteur minéralocorticoide humain. Les cellules obtenues sont lysées dans le tampon de lyse (4).

Le surnageant est incubé à 0°C pendant 24 heures avec une concentration constante (T) d'aldostérone tritiée en présence de concentrations croissantes soit d'aldostérone froide (de 0 à 1000 x $10^{-9}$M) soit du produit froid à tester (de 1 à 25000 x $10^{-9}$M). La concentration d'aldostérone tritiée liée (B) est ensuite mesurée dans chaque incubat par la méthode d'adsorption au charbon-dextran.

| **Tampons de lyse :** | |
|---|---|
| (1) Tris-HCl pH 8 | 20mM |
| EDTA | 0,5mM |
| KCl | 400mM |
| Glycérol | 20 % |
| Extemporanément, ajouter : | |
| DTT | 2mM |
| Mélange PIC* | 1 % |
| | |
| (2) $KH_2PO_4$, NaOH pH 7 | 50mM |
| Glycérol | 20 % |
| Extemporanément, ajouter : | |
| DTT (Dithiothreitol) | 5mM |
| Molybdate de sodium | 20mM |
| Mélange PIC* | 0,1 % |
| | |
| (3) Tris-HCl pH 7,5 | 20mM |
| EDTA, $2H_2O$ | 1mM |
| Glycérol | 10 % |
| Extemporanément, ajouter : | |
| PMSF (Phényl méthyl sulfonyl fluoride) | 0,1mM |
| Molybdate de sodium | 20mM |
| Mélange PIC* | 0,1 % |
| | |
| (4) Tris-HCl pH 7,4 | 20mM |
| EDTA, $2H_2O$ | 1mM |
| Glycérol | 10 % |
| Extemporanément, ajouter : | |
| Tungstate de sodium | 20mM |
| Mélange PIC* | 0,1 % |

* Mélange PIC : Leupeptine, Pepsatine A, Aprotinine, Anti-paine, Chymostatine (Chaque peptide est à la concentration finale de 2,5 µg/ml).

**Expression des résultats et méthodes de calcul**

- Calcul de l'affinité relative de liaison (ARL).

On trace les 2 courbes suivantes : pourcentage de l'hormone tritiée liée B/BO en fonction du logarithme de

la concentration de l'hormone de référence froide ou en fonction du logarithme de la concentration du produit froid testé.

On détermine la droite d'équation suivante :

$I_{50}$ = 100 ($B_0$/$B_0$+Bmin/$B_0$)/2 doit $I_{50}$ = 100 (1+Bmin/$B_0$)/2 = 50 (1+Bmin/$B_0$)
$B_0$ = Concentration de l'hormone tritiée liée en l'absence de tout produit froid,
B min = Concentration de l'hormone tritiée liée en présence de la concentration la plus élevée en hormone froide de référence.

Les intersections de la droite $I_{50}$ et des courbes, permettent d'évaluer les concentrations de l'hormone de référence froide (CH) et du produit froid testé (CX) qui inhibent de 50 % la liaison spécifique de l'hormone tritiée sur le récepteur.

L'affinité relative de liaison (ARL) du produit testé est déterminé par l'équation :

$$ARL = 100\ (CH)/(CX)$$

Les ARL des produits de référence Estradiol, Progestérone, Déxaméthasone, Testostérone et Aldostérone sont prises arbitrairement égales à 100.

Les résultats des ARL obtenus sont les suivants :

| Produits | Récepteur glucocorticoïde humain 24 h à 0°C Dexaméthasone = 100 | Récepteur progestogène humain 24 h à 0°C Progestérone = 100 | Récepteur androgène humain 24 h à 0°C Testostérone = 100 | Récepteur minéralocorticoïde humain 24 h à 0°C Aldostérone = 100 | Récepteur estrogène humain 24 h à 0°C Estradiol = 100 |
|---|---|---|---|---|---|
| Trimégestone | 14 | 588 | 2,5 | 28 | 0 |
| 1α-OH Produit A | 0,06 | 64 | 0,04 | 0,1 | 0 |
| 6β-OH Produit B | 0,05 | 12 | 0 | 0,3 | 0 |

Les produits dont l'ARL est égale à 0 ont une $IC_{50}$ supérieure à 25000 nM.

Conclusion :

Le produit A (1α-OH) présente une bonne affinité relative de liaison (ARL) pour le récepteur progestogène humain, bien que celle-ci soit 9 fois plus faible que celle de la Trimégestone. Par contre le produit B (6β-OH) ne présente qu'une ARL modérée pour ce récepteur.

Contrairement à la Trimégestone qui montre des ARL modérées pour les récepteurs glucocorticoide et minéralocorticoide humains, et une ARL faible pour le récepteur androgène humain, ces 2 produits ne présentent que des ARL négligeables ou nulles pour ces récepteurs.

Les ARL pour le récepteur estrogène humain, sont nulles pour la Trimégestone et les produits A et B.

2.- Détermination de l'activité progestomimétique des produits de l'invention : Transformation endométriale chez la lapine.

**a) Méthode.**

Les animaux utilisés sont des lapins femelles impubères âgées de 40-45 jours. Les animaux sont traités quotidiennement par de l'oestradiol (5 µg/0,2 ml/lapin en solution dans de l'huile de germe de maïs, 10% d'éthanol) par voie sous cutanée du jour 1 au jour 5, puis le produit à étudier est administré du jour 8 au jour 11. Au jour 12, les animaux sont sacrifiés, sur chaque animal, une portion médiane de chacune des cornes utérines est prélevée et fixée dans du liquide de Bouin pour une étude histologique. Après fixation, les échantillons sont déshydratés, inclus, coupés, montés, déparaffinés puis colorés à l'hémalun-Eosine-Safran.

**b) Histologie**

Les coupes sont examinées en microscopie optique, d'abord à faible grossissement, pour s'assurer qu'il n'existe pas de variations morphologiques de l'une à l'autre puis, pour chacune des cornes, une appréciation semi-quantitative de la prolifération endométriale (dentelle utérine) est effectuée selon l'échelle de Mc Phail de 0 à 4 avec des intermédiaires de 0,5 et les deux chiffres sont moyennés. De la même façon, le degré d'hypertrophie des cornes est évalué selon la même échelle. A plus fort grossissement, des obsrvations morphologiques sur la muqueuse ou le myomètre peuvent éventuellement être formulées.

**c) Protocole retenu.**

Le métabolite 1-OH (produit A) a été testé aux doses de 10, 100, 1000 µg/kg par voie sous cutanée ainsi que par voie orale à la dose de 1000 µg/kg. Le métabolite 6-OH (produit B) a été testé aux doses de 100, 1000 et 10000 µg/kg par voie sous cutanée. En contrôle, la progestérone a été administrée aux doses de 100 et 1000 µg/kg par voie sous cutanée.

**d) Références.**

<u>Clauberg, C.</u>

Zur Physiologie und Pathologie des Sexual Hormone, im besonderen des Hormons des Corpus Luteum 1. Mitt. : Der biologishe Test für das luteohormon am infantilen Kaninchen. Zentralbl. Gynakol. 1930, 54: 2757-70.

<u>Mc Phail, M.K.</u>

The assay of progestins
J. Physiol (London) 1934, 83: 145-56.
Remarque : la Trimegestone présente une activité progestomimétique, sur ce test, dès la dose de 1 µg/kg et une forte activité aux doses de 3 et 10 µg/kg par voie orale et 3 µg/kg par voie percutanée. D'autre part, l'activité maximale de la progestérone sur la dentelle utérine se situe à 1000 µg/kg par voie sous cutanée (indice Mc Phail de 4.).

<u>Résultats :</u>

| Essais | | Moyenne |
|---|---|---|
| HM + 10% ethanol, s/c | | 0 |
| Progestérone 0,1 mg/kg, s/c | | 1,8 |
| Progestérone 1 mg/kg, s/c | | 3,8 |
| Trimegestone 0,003 mg/kg, s/c | | 3,8 |
| Produit B | 0,01 mg/kg, s/c | 1,4 |
| Produit B | 0,1 mg/kg, s/c | 2 |
| Produit B | 1 mg/kg, s/c | 3,8 |
| Produit A | 0,01 mg/kg, s/c | 3,8 |
| Produit A | 0,1 mg/kg, s/c | 3,8 |
| Produit A | 1 mg/kg, s/c | 3,8 |
| Produit A | 1 mg/kg, per os | 4 |

Les moyennes sont réalisée pour la dentelle utérine (Indice Mac Phail).

**Conclusion :**

Ce test de Clauberg-Mac Phail montre une bonne activité progestomimétique du Produit B à 1 mg/kg (indice Mac Phail : 3,8).

Le Produit A est tout aussi actif dès la dose de 0,01 mg/kg (indice Mac Phail : 3,8) et à 1 mg/kg la voie p/o exprime une meilleure activité que s/c (indice Mac Phail : 4 versus 3,8 et plus forte hypertrophie des cornes).
Par ailleurs, l'observation histologique des coupes d'utérus ne révèle pas d'anomalies morphologiques.

**Revendications**

1. Les composés de formule générale (I) :

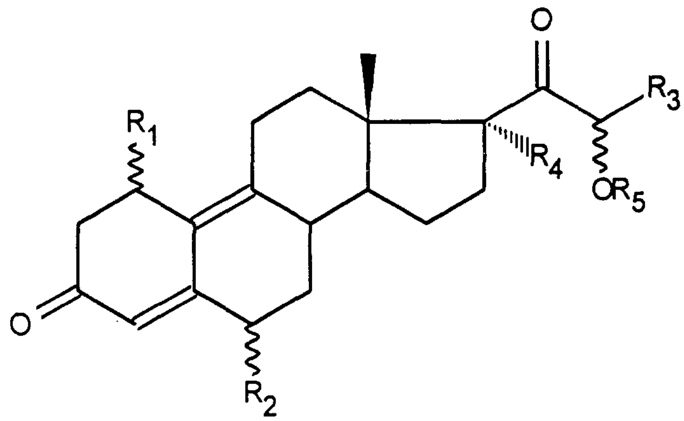

(I)

dans laquelle :

soit $R_1$ est un atome d'hydrogène et $R_2$ est un radical $OR_6$,
soit $R_1$ est un radical $OR_6$ et $R_2$ est un atome d'hydrogène, $R_6$ représentant un radical acyle renfermant de 1 à 12 atomes de carbone, éventuellement substitué par un ou plusieurs atomes d'halogènes, ou un atome d'hydrogène,
$R_3$ est un radical alkyle renfermant de 1 à 12 atomes de carbone,
$R_4$ est un radical alkyle renfermant de 1 à 4 atomes de carbone et
$R_5$ identique ou différent de $R_6$, possède les mêmes valeurs que $R_6$, les traits ondulés indiquant que $R_1$ ou $R_2$ sont en position $\alpha$ ou $\beta$ et $OR_5$ est en position 21R ou 21S.

2. Les composés de formule (I) telle que définie à la revendication 1, dans laquelle $R_3$ et $R_4$ sont des radicaux méthyle.

3. Les composés de formule (I) telle que définie à la revendication 1, dans laquelle $R_1$ est un atome d'hydrogène et $R_2$ est un groupement hydroxyle.

4. Les composés de formule (I) telle que définie à la revendication 1, dans laquelle $R_1$ est un groupement hydroxyle et $R_2$ est un atome d'hydrogène.

5. Composés de formule (I) selon l'une quelconque des revendications 1 à 4, dont les noms suivent :

- [17β-(S)]-1-hydroxy-17-(2-hydroxy-1-oxopropyl)-17-méthyl estra-4,9-dièn-3-one,
- [17β-(S)]-6-hydroxy-17-(2-hydroxy-1-oxopropyl)-17-méthyl estra-4,9-dièn-3-one,

sous l'une quelconque de leur configuration stéréoisomère 1α-OH, 1β-OH, 6α-OH, 6β-OH, seuls ou en mélange.

6. Les composés de formule (I) telle que définie à la revendication 1, associés à un composé estrogène.

7. Les composés tels que définis à la revendication 5, associés avec le 17β-estradiol.

8. Procédé de préparation des composés de formule générale (I) tels que définis à la revendication 1, **caractérisé en ce qu'**on effectue une bioconversion du composé de formule générale (II) :

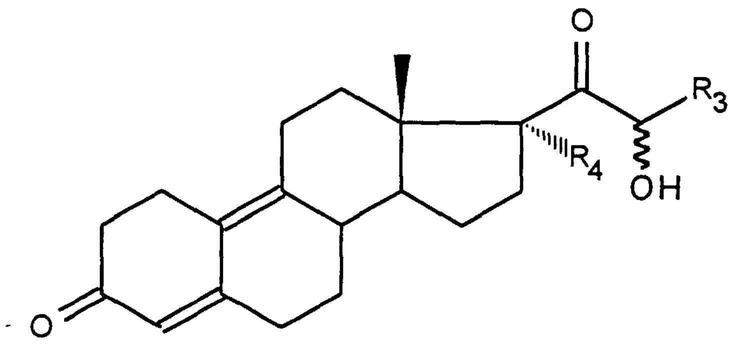

(II)

dans laquelle $R_3$, $R_4$ et les traits ondulés ont les mêmes définitions que celles données précédemment, par une culture de champignons filamenteux, puis le cas échéant on procède à une séparation des produits 1-hydroxylés et 6-hydroxylés et/ou à une estérification des groupements hydroxyles libres.

**9.** Procédé selon la revendication 8, **caractérisé en ce que** le champignon filamenteux est choisi parmi les souches suivantes :

| | |
|---|---|
| Cuninghamella baineri | ATTC 9244 |
| Mortierella isabellina | NRLL 1757 ou MMP 108 |
| Fusarium roseum | ATCC 14717. |

**10.** Procédé selon la revendication 8, **caractérisé en ce que** le produit de formule (II) est la Trimégestone :

- [17β-(S)]-17-(2-hydroxy-1-oxopropyl)-17-méthyl-estra-4,9-dièn-3-one.

**11.** Procédé selon la revendication 8, **caractérisé en ce que** le champignon filamenteux est le :
Cuninghamella baineri, le Mortierella isabellina ou le Fusarium roseum,
et le produit de formule (II) est la Trimégestone, afin d'obtenir les dérivés 1-hydroxylé et 6-hydroxylé :

- [17β-(S)]-1-hydroxy-17-(2-hydroxy-1-oxopropyl)-17-méthyl estra-4,9-dièn-3-one,
- [17β-(S)]-6-hydroxy-17-(2-hydroxy-1-oxopropyl)-17-méthyl estra-4,9-dièn-3-one

qui sont par la suite séparés.

**12.** Procédé selon la revendication 8, **caractérisé en ce que** le champignon filamenteux est le Cuninghamella baineri ATCC 9244,
et le produit de formule (II) est la Trimégestone,
afin d'obtenir le dérivé 1-hydroxylé :

- [1α,17β-(S)]-1-hydroxy-17-(2-hydroxy-1-oxopropyl)-17-méthyl estra-4,9-dièn-3-one ou [1β,17β-(S)]-1-hydroxy-17-(2-hydroxy-1-oxopropyl)-17-méthyl estra-4,9-dièn-3-one.

**13.** A titre de médicament, les composés de formule générale (I) telle que définie à l'une quelconque des revendications 1 à 4.

**14.** A titre de médicament, les composés de formule générale (I) tels que définis à la revendication 5.

**15.** A titre de médicaments les associations telles que définies à l'une quelconque des revendications 6 et 7.

**16.** Les compositions pharmaceutiques renfermant comme principe actif au moins l'un des médicaments tels que définis à l'une quelconque des revendications 13 à 15.

**Patentansprüche**

1. Verbindungen der allgemeinen Formel (I)

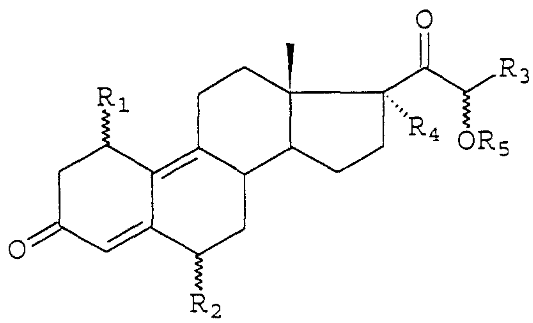

(I)

in der

entweder $R_1$ ein Wasserstoffatom ist und $R_2$ einen Rest $OR_6$ darstellt,
oder $R_1$ einen Rest $OR_6$ bedeutet und $R_2$ ein Wasserstoffatom ist, wobei $R_6$ einen Rest Acyl mit 1 bis 12 Kohlenstoffatomen, gegebenenfalls substituiert durch ein oder mehrere Halogenatome, oder ein Wasserstoffatom darstellt,
$R_3$ einen Rest Alkyl mit 1 bis 12 Kohlenstoffatomen bedeutet,
$R_4$ einen Rest Alkyl mit 1 bis 4 Kohlenstoffatomen darstellt und
$R_5$, identisch mit oder verschieden von $R_6$, die gleichen Werte wie $R_6$ besitzt, wobei die wellenförmigen Bindungen anzeigen, daß sich $R_1$ oder $R_2$ in Position $\alpha$ oder $\beta$ und $OR_5$ in Position 21R oder 21S befinden.

2. Verbindungen der Formel (I) wie in Anspruch 1 definiert, worin $R_3$ und $R_4$ Reste Methyl sind.

3. Verbindungen der Formel (I) wie in Anspruch 1 definiert, worin $R_1$ ein Wasserstoffatom ist und $R_2$ eine Gruppe Hydroxyl darstellt.

4. Verbindungen der Formel (I) wie in Anspruch 1 definiert, worin $R_1$ eine Gruppe Hydroxyl ist und $R_2$ ein Wasserstoffatom darstellt.

5. Verbindungen der Formel (I) nach irgendeinem der Ansprüche 1 bis 4 mit den folgenden Namen:

- [17β-(S)]-1-Hydroxy-17-(2-hydroxy-1-oxopropyl)-17-methyl-estra-4,9-dien-3-on,
- [17β-(S)]-6-Hydroxy-17-(2-hydroxy-1-oxopropyl)-17-methyl-estra-4,9-dien-3-on,

in irgendeiner ihrer stereoisomeren Konfiguration 1α-OH, 1β-OH, 6α-OH, 6ß-OH, allein oder in Mischung.

6. Verbindungen der Formel (I) wie in Anspruch 1 definiert, assoziiert mit einer estrogenen Verbindung.

7. Verbindungen der Formel (I) wie in Anspruch 1 definiert, assoziiert mit 17β-Estradiol.

8. Verfahren zur Herstellung von Verbindungen der allgemeinen Formel (I) wie in Anspruch 1 definiert, **dadurch gekennzeichnet, daß** man eine Biokonversion bei einer Verbindung der allgemeinen Formel (II)

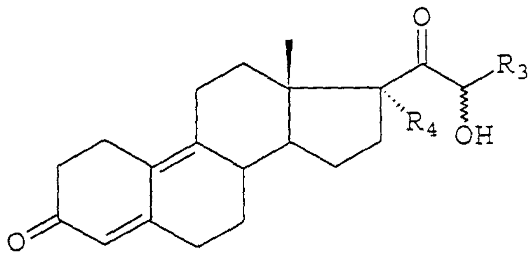

(II)

in der $R_3$, $R_4$ und die wellenförmigen Bindungen die gleichen Definitionen wie vorstehend angegeben besitzen, mit Hilfe einer Kultur von Fadenpilzen durchführt, anschließend gegebenenfalls eine Trennung der 1-hydroxylierten und der 6-hydroxylierten Produkte und/oder eine Veresterung der freien Gruppen Hydroxyl vornimmt.

**9.** Verfahren nach Anspruch 8, **dadurch gekennzeichnet, daß** der Fadenpilz unter den folgenden Stämmen ausgewählt wird:

| | |
|---|---|
| Cuninghamella baineri | ATCC 9244 |
| Mortierella isabellina | NRLL 1757 oder MMP 108 |
| Fusarium roseum | ATCC 14717. |

**10.** Verfahren nach Anspruch 8, **dadurch gekennzeichnet, daß** das Produkt der Formel (II) Trimegeston ist:

- [17β-(S)]-17-(2-Hydroxy-1-oxopropyl)-17-methyl-estra-4,9-dien-3-on.

**11.** Verfahren nach Anspruch 8, **dadurch gekennzeichnet, daß** der Fadenpilz Cuninghamella baineri, Mortierella isabellina oder Fusarium roseum ist und das Produkt der Formel (II) Trimegeston ist, um die folgenden 1-hydroxylierten und 6-hydroxylierten Derivate zu erhalten:

- [17β-(S)]-1-Hydroxy-17-(2-hydroxy-1-oxopropyl)-17-methyl-estra-4,9-dien-3-on,
- [17β-(S)]-6-Hydroxy-17-(2-hydroxy-1-oxopropyl)-17-methyl-estra-4,9-dien-3-on,

die später getrennt werden.

**12.** Verfahren nach Anspruch 8, **dadurch gekennzeichnet, daß** der Fadenpilz Cuninghamella baineri ATCC 9244 ist und das Produkt der Formel (II) Trimegeston ist, um das 1-hydroxylierte Derivat zu erhalten:

- [1α,17β-(S)]-1-Hydroxy-17-(2-hydroxy-1-oxopropyl)-17-methyl-estra-4,9-dien-3-on oder
- [1β,17β-(S)]-1-Hydroxy-17-(2-hydroxy-1-oxopropyl)-17-methyl-estra-4,9-dien-3-on.

**13.** Als Arzneimittel die Verbindungen der allgemeinen Formel (I) wie in irgendeinem der Ansprüche 1 bis 4 definiert.

**14.** Als Arzneimittel die Verbindungen der allgemeinen Formel (I) wie in Anspruch 5 definiert.

**15.** Als Arzneimittel die Assoziationen wie in irgendeinem der Ansprüche 6 und 7 definiert.

**16.** Pharmazeutische Zusammensetzungen, umfassend als Wirkstoff mindestens eines der Arzneimittel wie in irgendeinem der Ansprüche 13 bis 15 definiert.

## Claims

**1.** The compounds of general formula (I) :

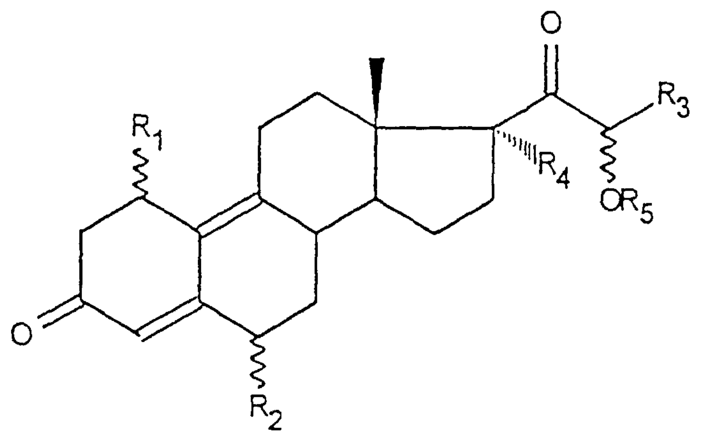

(I)

in which:

<u>either</u> $R_1$ is a hydrogen atom and $R_2$ is an $OR_6$ radical,
<u>or</u> $R_1$ is an $OR_6$ radical and $R_2$ is a hydrogen atom, $R_6$ representing an acyl radical containing 1 to 12 carbon atoms, optionally substituted by one or more halogen atoms, or a hydrogen atom,
$R_3$ is an alkyl radical containing 1 to 12 carbon atoms,
$R_4$ is an alkyl radical containing 1 to 4 carbon atoms and
$R_5$ identical to or different from $R_6$, has the same values as
$R_6$, the wavy lines indicating that $R_1$ or $R_2$ are in $\alpha$ or $\beta$ position and $OR_5$ is in 21R or 21S position.

**2.** The compounds of formula (I) as defined in claim 1, in which $R_3$ and $R_4$ are methyl radicals.

**3.** The compounds of formula (I) as defined in claim 1, in which $R_1$ is a hydrogen atom and $R_2$ is a hydroxyl group.

**4.** The compounds of formula (I) as defined in claim 1, in which $R_1$ is a hydroxyl group and $R_2$ is a hydrogen atom.

**5.** Compounds of formula (I) according to any one of claims 1 to 4, the names of which follow:

- [17β-(S)]-1-hydroxy-17-(2-hydroxy-1-oxopropyl)-17-methyl estra-4,9-dien-3-one,
- [17β-(S)]-6-hydroxy-17-(2-hydroxy-1-oxopropyl)-17-methyl estra-4,9-dien-3-one,

in any one of their 1α-OH, 1β-OH, 6α-OH, 6β-OH stereoisomer configuration, alone or in a mixture.

**6.** The compounds of formula (I) as defined to claim 1, combined with an estrogen compound.

**7.** The compounds as defined in claim 5, combined with 17β-estradiol.

**8.** Process for the preparation of the compounds of general formula (I) as defined in claim 1, **characterized in that** a bioconversion of the compound of general formula (II):

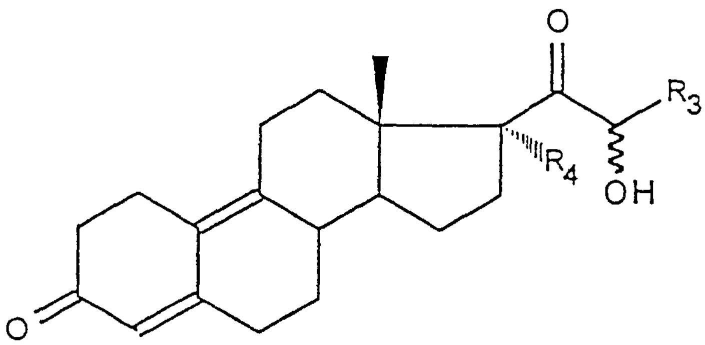

(II)

in which $R_3$, $R_4$ and the wavy lines have the same definitions as those given previously, is carried out by a culture of filamentous fungi, then if appropriate, separation of the 1-hydroxylated and 6-hydroxylated products is carried out and/or esterification of the free hydroxyl groups.

**9.** Process according to claim 8, **characterized in that** the filamentous fungi is chosen from the following strains:

| | |
|---|---|
| Cuninghamella baineri | ATTC 9244 |
| Mortierella isabellina | NRLL 1757 or MMP 108 |
| Fusarium roseum | ATCC 14717. |

**10.** Process according to claim 8, **characterized in that** the product of formula (II) is Trimegestone:

- [17β-(S)]-17-(2-hydroxy-1-oxopropyl)-17-methyl-estra-4,9-dien-3-one.

**11.** Process according to claim 8, **characterized in that** the filamentous fungi is:
Cuninghamella baineri, Mortierella isabellina or Fusarium roseum,
and the product of formula (II) is Trimegestone,
in order to obtain the 1-hydroxylated and 6-hydroxylated derivatives:

- [17β-(S)]-1-hydroxy-17-(2-hydroxy-1-oxopropyl)-17-methyl estra-4,9-dien-3-one,
- [17β-(S)]-6-hydroxy-17-(2-hydroxy-1-oxopropyl)-17-methyl estra-4,9-dien-3-one

which are then separated.

**12.** Process according to claim 8, **characterized in that** the filamentous fungi is Cuninghamella baineri ATCC 9244, and the product of formula (II) is Trimegestone,
in order to obtain the 1-hydroxylated derivative:

- [1α,17β-(S)]-1-hydroxy-17-(2-hydroxy-1-oxopropyl)-17-methyl estra-4,9-dien-3-one or [1β,17β-(S )]-1-hydroxy-17-(2-hydroxy-1-oxopropyl)-17-methyl estra-4,9-dien-3-one.

**13.** As a medicament, the compounds of general formula (I) as defined in any one of claims 1 to 4.

**14.** As a medicament, the compounds of general formula (I) as defined in claim 5.

**15.** As a medicaments the combinations as defined in any one of claims 6 and 7.

**16.** The pharmaceutical compositions containing as active ingredient at least one of the medicaments as defined in any one of claims 13 to 15.